Europäisches Patentamt

European Patent Office

Office européen des brevets

(11) Publication number: **0 535 663 A2**

## EUROPEAN PATENT APPLICATION

(21) Application number: **92116788.8**

(22) Date of filing: **01.10.92**

(51) Int. Cl.⁵: **C12P 21/08**, G01N 33/574, G01N 33/543

(30) Priority: **02.10.91 IL 99630**

(43) Date of publication of application:
**07.04.93 Bulletin 93/14**

(84) Designated Contracting States:
**BE DE FR GB IT**

(71) Applicant: **Technion Research & Development Foundation Ltd.**
**Technion City**
**IL-32000 Haifa(IL)**

(72) Inventor: **Shalittin, Channa**
**70, Pinsker St.**
**Haifa(IL)**

(74) Representative: **Portal, Gérard et al**
**Cabinet Beau de Loménie 158, rue de l'Université**
**F-75340 Paris Cédex 07 (FR)**

(54) **Novel antibodies useful for the detection of benign and malignant tumors.**

(57) Novel anti-non-**ras**-p21 antibodies with a particular N-terminal aminoacids sequence of two peptides derived from the non-**ras**-p21 protein are described. These antibodies were found to be able to detect malignant diseases by their reaction with biological fluids of patients with tumors. The method for this detection uses a modified ELISA procedure. This ELISA procedure with the novel anti-non-**ras**-p21 antibodies were found to be useful for clinical monitoring of patients with different types of tumors, such as urogenital and gastrointestinal, as well as malignant lymphomas, some of which have no available tumor marker.

EP 0 535 663 A2

The present invention relates to novel anti-non-**ras**-p21 antibodies. More particularly, the invention relates to a method for the detection of non-**ras**-p21 protein in biological fluids of benign and malignant tumor bearing patients, using a modified **E**nzyme-**L**inked **I**mmunosorbent **A**ssay (ELISA) procedure.

## BACKGROUND OF THE INVENTION

The release of antigenic compounds from tumor cells into the blood circulation as a result of cell death has been described in a paper by Herlyn et al (Advances in Cancer Res., 49, 189-221, 1987). These antigenic substances may, serve as tumor markers. Markers of disease activity in sera of patients with prostate cancer include prostatic acid phosphatase and Prostate Specific Antigen (PSA). PSA is made exclusively by prostatic tissue and it is immunologically and biochemically distinct from prostatic acid phosphatase. An assay was developed by Stamey and Kabalin (J.Urol. 141, 1070-1075, 1989) to detect PSA in serum, showing that it was directly proportional to the clinical stage. It was shown that PSA was also strongly correlated with the volume of prostate cancer.

Another tumor antigen named CA 125, common to most non-mucinous epithelial ovarian carcinomas, was described by Sulizeanu (advances in Cancer Res., 44,1-42, 1985). Concentrations of CA 125 were found to be low in the sera of all healthy individuals and high in very few patients with benign disease, but very high in the sera of a majority of patients with ovarian carcinomas (Klug et.al. Cancer Res. 44, 1048-1053, 1984).

The monitoring of the course of epithelial ovarian cancer using the radioimmunoassay procedure, is described in a paper by R.C.Bast et.al.(The New England Journal of Medicine, Vol. 309, No.15, p.883-887, 1983). As mentioned therein, the murine monoclonal immunoglobulin (OC 125) reacts with the antigen CA 125. Determination of the CA 125 levels is mentioned to be useful in monitoring the response to treatment in patients with epithelial ovarian cancer.

The concentrations of serum **p**rostate **s**pecific **a**ntigen (PSA), to be used as a serum marker were determined using $^{125}$I in the common radioimmunoassay procedure (T.A.Stamey et.al., The Journal of Urology, 1088-90, 1989). Although the method has its merits due to the reliable results, it has the disadvantage that it requires the costly radioisotopes among the other well-known drawbacks of these methods such as disposal of radioisotopes. The detection of a 21kDa protein in sera of patients with urogenital tumors, was recently described by Shalitin et.al, (The Cancer J., 3, 74-78, 1990). As mentioned therein, the serum level of this protein can not be used as a differential diagnostic tumor marker for malignancy, since it was found that there is a high level of p21 in the sera of patients with benign hyperplasia of the prostate (BPH).

In another recent paper (A.Kakkamas & Spandidos, In-vivo 4, 115-120, 1990) the presence of ras p21 onco-protein in the sera of mice carrying experimentally induced tumors, was determined by the standard ELISA procedure. According to this procedure,the original serum was inserted in common microtiter plates and washed with a gelatin solution. Monoclonal antibody Y13-259 was added at 1/300 dilution in gelatin solution and incubated at 4°C for 18 hours. The plate was then washed three times with PBS containing 0.5% Tween 20. The second antibody, goat anti-rat IgG (from Sigma) was added at 1/1000 dilution in gelatin solution at 37°C for 2 hours, followed by two washes with PBS containing 0.5% Tween 20. Finally, streptavidin-peroxidase (from Sigma) was added at 1/1000 dilution in gelatin solution at 37°C for 2 hours,and the plate was washed with PBS containing 0.5% Tween 20 and dried. After the final wash the reaction was developed with a substrate solution:

24.2 mM citric acid monohydrate, 58.65 mM $NaH_2PO_4.2H_2O$, 0.015% $H_2O_2$ and 50 $\mu$g/ml orthophenyl-diamine (O.P.D., from Sigma). The reaction was stopped by the addition of 3N HCl and the optical density (O.D.) was measured at 492 nm with a Titertec photometer (from Flow).

A serum immunoblotting technique based on monoclonal antibodies, to peptide sequences of **ras** oncogene-related protein (p21) was recently described (Brandt-Rauf et al, Int.J.Cancer 50, 881-885, 1992).

It is an object of the present invention to provide novel anti-non-**ras**-p21 antibodies, to be used for the detection of benign and malignant tumors. It is another object of the present invention, to provide a new method for the monitoring of patients with different types of tumors such as urogenital and gastrointestinal tumors as well as malignant lymphomas, using said anti-non-**ras**-p21 antibodies with a modified ELISA procedure. It is yet another object of the present invention to provide an immunoblotting and immunoprecipitation procedure for the detection of non-**ras**-p21.

## BRIEF DESCRIPTION OF THE INVENTION.

The invention relates to new anti-non-**ras**-p21 antibodies to be useful for the detection of benign and malignant tumors in biological fluids of patients with tumors which comprises the steps of: (a) isolation Of the 21K Dalton protein from a biological fluid; (b) preparation of anti-non-**ras**-p21 polyclonal antibodies which do not possess immunoreactivity with other biological fluid proteins and (c) determining the total non-**ras**-p21 concentration in the biological fluids using a modified ELISA procedure. By monitoring the non-**ras**-p21 levels of patients with various malignant diseases, pre-treatment and post-treatment during the follow-up, it is possible to get a clinically useful marker of progression or regression of the disease and thus could provide evidence of response to the applied treatment.

## BRIEF DESCRIPTION OF THE FIGURES.

**Figure 1,**    illustrates the immunoreactivity of anti-non-**ras**-p21 antibodies with non-**ras** p21.

**Figure 2,**    shows no cross-reactivity of anti-non-**ras**-p21 antibodies with bacterially expressed $C^H$**ras**.

**Figure 3,**    illustrates the linearity of the calibration curve using the anti-non-**ras**-p21 rabbit antibodies.

**Figure 4,**    compares the values of non-**ras** p21 levels in sera from patients with benign prostatic hypertrophy (BPH) compared with those of patients with various malignant diseases and healthy donors.

**Figure 5,**    illustrates the effect of therapy on the level of non-**ras**-p21 in sera of patients with urogenital diseases.

**Figure 6,**    shows the level of serum non-**ras**-p21 before and after radical unilateral nephrectomy in patients with renal cell carcinoma.

**Figure 7,**    illustrates the clinical course of 63 year old man with stage C prostate cancer. Sequential influence on serum non-**ras**-p21 as compared with PSA of hormone therapy (H) and radiotherapy (RT) are shown.

## DETAILED DESCRIPTION OF THE INVENTION AND FIGURES.

The p21 was identified by a treatment which comprises the cleaving of the protein with cyanogen bromide and the peptides separated by reversed phase chromatography. It was found that the non-**ras**-p21, has a molecular weight of 21KDa and possesses the following N-terminal amino-acid sequence of two peptides derived from the non-**ras**-p21 protein:

```
Peptide No.1 (SEQ ID NO:1)
Ala Val Gly Asp Arg Arg His Glu Arg Asn Glu Phe Gln Gln Gln
1           5                 10              15
```

```
Peptide No.2 (SEQ ID NO:2)
Ala Val Ser Pro Ser Pro Thr Phe Leu Thr Gln Gln
1           5                 10
```

The repetitive yield of the above amino acids was calculated to be above 95% which is considered a very high yield for a new protein.

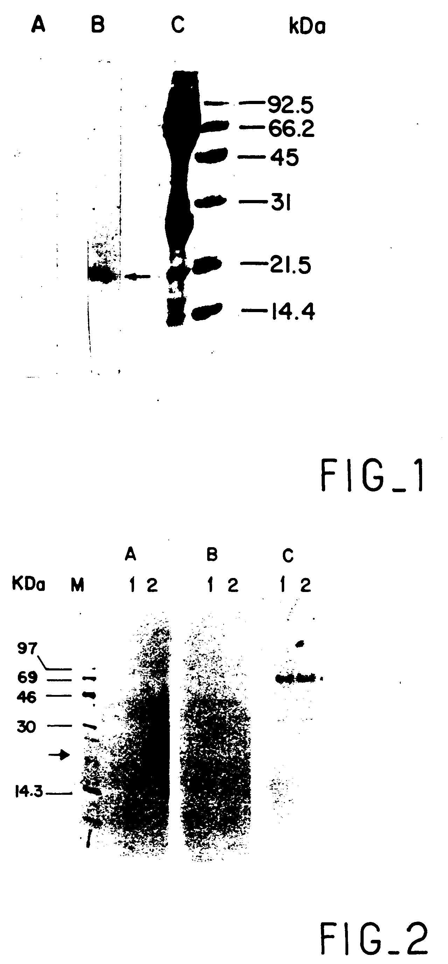

In the first step, the non-**ras**-p21 was isolated from sera of patients with urogenital tumors as well as from healthy persons, used as control. A full description of this step will be further described in a detailed manner. The specificity of anti-non-**ras**-p21 antibodies is illustrated in Figure 1. The specificity of the rabbit polyclonal antibodies was tested using Western blots of immune complexes or non-**ras**-p21 immunoglobulin adducts (formed by disulfide bonds between the two proteins). As can be noticed from this Figure, the antibodies detected only a single non-**ras**-p21 band. No immunoreactivity of the antibodies with other serum proteins was detected.

The p21 protein was isolated from sera of patients with urogenital tumors. Following polyacrylamide-SDS gel electrophoresis, the Coomassie stained p21 was excised and eluted from SDS-PAGE gels (using Model 422 Electro-Eluter, BioRad) at 8-10mA/glass tube for 90 minutes at room temperature in a protein-elution buffer (Tris base 20mM glycine 192mM 0.1% SDS W/V). The eluted protein was precipitated with chloroform and methanol. Using this procedure the Coomassie stain as well as the SDS were removed. Dried protein pellet was kept at -20°C and used for immunizing rabbits.

The specificity or the rabbit polyclonal antibodies to non-**ras**-p21 in contrast to genuine human **ras**-p21, was tested. The results which were obtained are presented in the Figure 2, wherein it clearly appears that the anti-non-**ras** antibodies do not immunoreact with bacterially expressed c**H**ras. Cells of an overnight culture of RRIΔM15 in LB medium, or ptac c**H**ras in RRIΔM15 in LB medium containing 100μg/ml ampicillin were collected and the cells were lysed. Aliquots of 5μl of this solution were separated on a 15% polyacrylamide-SDS gel. Proteins were transferred onto nitrocellulose membrane and used for immunoblotting.

An alternative procedure for the isolation of the non-**ras**-p21, is by resolving the proteins on a 15% (by wt) polyacrylamide sodium dodecyl sulfate (SDS) gel under reducing conditions and further electroblotting the proteins from the gel onto membranes of polyvinylidene difluoride Immobilon (Trade Mark produced by Millipore Corporation) in the presence of a buffer containing 10mM (cyclohexylamino)-1-propanesulfonic acid, 10% methanol, pH 11.0. The membrane was stained with 0.1% Coomassie blue R in 50% methanol for 1 min. The stained 21kDa protein band was excised with a razor blade. Approximately 100-150 pmoles of the protein were eluted from the membrane using a volatile cocktail containing 30% TFA and 40% acetonitrile.After evaporation in a speed vac centriifuge, the sample was sent to Integrated Protein Technologies (Toronto, Canada) for protein sequence analysis. The sample was dissolved in 100μul of 70% formic acid, and 10μul cyanogen bromide (40 mg/ml CNBr in 70% formic acid) were added. Digestion was carried out at room temperature for 18 hours in dark. Excess CNBr and formic acid was removed under nitrogen, and the sample was lyophilized. The peptides were resolved by reverse-phase high-performance chromatography. The sample in 100μul of 0.1% trifluoroacetic acid was loaded onto a 4 x 100 mm cartridge containing a C18 Silica-based reverse-phase support.A gradient of 0-100% solvent B was applied to the column, where solvent B was 80% acetonitrile, 0.072% TFA in MiliQ water.Four majors peaks at 214 nm were resolved. One of the peaks was subjected to protein sequence analyysis using an automated gas-phase sequencer (Applied Biosystems, Focter City, CA; model 470A).

The last step of the method using a modified ELISA procedure is most critical for the present invention, enabling the detection of the non-**ras**-p21 protein in biological fluids of benign and malignant tumor bearing patients. This procedure is characterized by the following particular features which are not encountered in the normal ELISA procedure:

- The serum from the cancer patients is heated at a temperature above 50°C, in order to inactivate the complement, or an interference factor which may interfere with the procedure. Generally the heating period is for about 30 minutes at 56°C. This step has a remarkable enhancing effect on the signal obtained.
- The human serum derived non-**ras**-p21 protein is used in a calibration curve for polyclonal antibodies generated against this product. The calibration curve should be linear from zero to 150 ng/ml as compared with a range of zero to 50 ng/ml for the radioimmunoassay of p-PSA assay (Pros-Check, Trade Mark, produced by Yang Lab. Inc.,Bellevue) or zero to 100 ng/ml for M-PSA assay in a solid-phase two site immunoradiometric assay (produced by Hybritech Inc., San Diego).
- The microtiter plate has to be blocked with a 2% (by volume) low fat milk in phosphate bufferred saline (PBS) and not with gelatin as commonly used. In this manner, the background will be low, in contrast to a higher one which is obtained with gelatin. Preferably, the anti-non-**ras**-p21 antibodies may also be diluted with the same solution of PBS containing 2% low fat milk.
- The human serum has to be diluted in a coating buffer with a pH of 9.6 containing sodium carbonate and sodium bicarbonate in the presence of PMSF (phenyl methyl sulfonyl fluoride). It was found that in the absence of the PMSF, the amount of protein bound to the plate decreased significantly.
- The reaction should be stopped by the addition of sulfuric acid (2N) rather than hydrochloric acid (3N) as generally used and described by Kakkamas et al (In-vivo 4,115-20, 1990) for the standard ELISA procedure. It was unexpectedly found that the use of a solution of hydrochloric acid, did not stop the reaction.

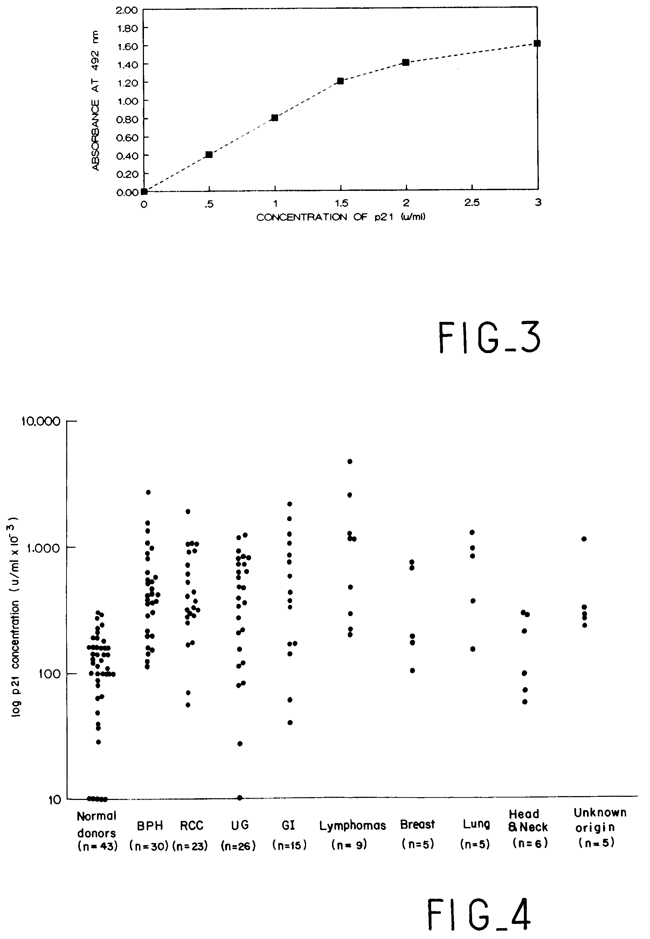

A panel of sera from cancer patients, or patients with benign hyperplasia of the prostate, was used to evaluate the results obtained with the modified ELISA procedure according to the present invention,using the anti-non-**ras**-p21 rabbit antibodies. The linearity of the calibration curve from 0 to 1.5 units/ml is presented in the Figure 3. It can be noticed that 1μl of human serum is enough for the detection of a tumor. Non-**ras**-p21 concentrations as low as 10,000 arbitrary units/ml can be detected.

Figure 4 presents the values of p21 levels in sera from patients with **b**enign **p**rostatic **h**ypertrophy **(BPH)** compared with those of patients with renal cell carcinoma urogenital cancer, gastro-intestinal cancer, breast and lung cancer, malignant lymphomas, head and neck cancer and healthy donors. The clinical characteristics of the cancer patients are shown in the following Table 1.

## Table 1: Clinical characteristics of cancer patients.

### Sex

| Male | 68 |
| --- | --- |
| Female | 26 |

### Age (years)

| Mean ± S.D | 63.5 ± 12.4 |
| --- | --- |
| Range | 21-86 |

| Site of tumor. | Number of patients. |
| --- | --- |
| Kidney | 23 |
| Prostate | 16 |
| Urinary Bladder | 7 |
| Ovary | 1 |
| Testis | 2 |
| Stomach | 5 |
| Colon | 5 |
| Pancreas | 3 |
| Liver | 2 |
| Non-Hodkgin's Lymphoma | 7 |
| Hodkgin's Lymphoma | 2 |
| Breast | 5 |
| Lung | 5 |
| Vocal cord | 3 |
| Larynx | 2 |
| Nasopharynx | 1 |
| Unknown origin | 5 |

Significant increased levels of non-**ras** p21 were found in patients with BPH and in patients with cancer. As appears from the Figure 4, the mean concentration was $571 \pm 543 \times 10^{-3}$ arbitrary units/ml in patients with BPH and $1358 \pm 1497 \times 10^{-3}$ u/ml in patients with malignant lymphomas. In healthy persons low levels with a mean concentration of only $123\pm77\times10^{-3}$ u/ml can be noticed. The results are summarized in the following Table 2.

## TABLE 2: SUMMARY OF p21 SERUM LEVELS BY ELISA ASSAYS.

|              | Normal  | BPH       | RCC      | UG       | GI       |
|--------------|---------|-----------|----------|----------|----------|
| N tested     | 43      | 30        | 23       | 26       | 15       |
| mean ± S.D.* | 123±77  | 571±543   | 541±437  | 475±351  | 666±633  |
| median*      | 128     | 405       | 368      | 427      | 437      |
| range*       | 10-300  | 115-2736  | 57-1920  | 10-1225  | 40-2140  |
| N(%)positive** | 2(4.6) | 22(73.3) | 18(78.2) | 16(61.5) | 10(66.6) |

|              | Lymph-omas  | Breast   | Lung      | Head & Neck | Unknown Origin |
|--------------|-------------|----------|-----------|-------------|----------------|
| N tested     | 9           | 5        | 5         | 6           | 5              |
| mean ± S.D.* | 1358±1497   | 375±307  | 680±398   | 167±105     | 420±331        |
| median*      | 1152        | 190      | 832       | 151         | 279            |
| range*       | 194-4800    | 106-742  | 153-1059  | 58-288      | 230-1011       |
| N(%)positive** | 7(77.7)   | 2(40)    | 4(80)     | 2(33.3)     | 3(60)          |

\*  expressed in u/ml x $10^{-3}$.

\*\* Serum p21 level higher than mean + 2 S.D. of normal group ($277\times10^{-3}$ u/ml).

BPH = benign hyperplasia of the prostate.

RCC = renal cell carcinoma.

UG  = urogenital cancer.

GI  = gastro-intestinal.

It was found that in 73.3% of patients with BPH and in 78.2% with malignant kidney tumors, the serum non-**ras**-p21 was significantly elevated (2 to 5 fold) with values greater than the mean plus 2 S.D of the norml group ($277\times 10^{-3}$u/ml).

In the following Table 3, are presented the results which show the high levels of serum p21 associated with benign prostatic hyperplasia in patients undergoing retropubic prostatectomy (the data are mean±SD of

2 determinations).

TABLE 3: Preoperative serum p21 levels in patients with benign prostatic hypertrophy[1] in relation to tumor mass.

| Case No. | Age | Tumor[2] mass (g) | Serum p21 levels[3] | |
|---|---|---|---|---|
| | | | A | B |
| 1 | 76 | 45 | $1360 \pm 240$ | $30.2 \pm 5.3$ |
| 2 | 69 | 40 | $400 \pm 96$ | $10.0 \pm 2.4$ |
| 3 | 71 | 40 | $410 \pm 10$ | $10.2 \pm 0.2$ |
| 4 | 79 | 105 | $985 \pm 25$ | $9.3 \pm 0.2$ |
| 5 | 69 | 65 | $575 \pm 35$ | $8.8 \pm 0.5$ |
| 6 | 72 | 65 | $368 \pm 32$ | $5.6 \pm 0.5$ |
| 7 | 79 | 75 | $415 \pm 65$ | $5.5 \pm 0.9$ |
| 8 | 69 | 130 | $549 \pm 19$ | $4.2 \pm 0.1$ |
| 9 | 78 | 110 | $465 \pm 15$ | $4.2 \pm 0.1$ |
| 10 | 78 | 175 | $535 \pm 105$ | $3.0 \pm 0.6$ |
| 11 | 79 | 120 | $215 \pm 25$ | $1.8 \pm 0.2$ |

(1) All patients with benign prostatic hypertrophy were confirmed pathologically. Patients underwent retro-pubic prostatectomy.

(2) Tumor mass was determined by weight.

(3) Serum p21 levels were assayed by the ELISA method.

A, u/mlx10$^{-3}$ data are mean$\pm$SD of 2 determinations.

B, u/mlx10$^{-3}$ per gram of hyper. tissue. The mean ELISA value$\pm$SD was divided by tumor weight.

As can be noticed from the above Table, in 4 out of 11 patients (cases Nos. 2 to 5) which underwent retropubic prostatectomy, serum non-**ras**-p21 levels correlated with the size of the tumor yielding 8,800-10,000 u/ml per gram of hyperplastic tissue. In 4 other patients (cases Nos. 6 to 9) a level of 4,200 to 5,600 u/ml per gram of hyperplastic tissue was assessed. This may indicate a lower level of non-**ras**-p21 expression in the hyperplastic tissue of some patients.

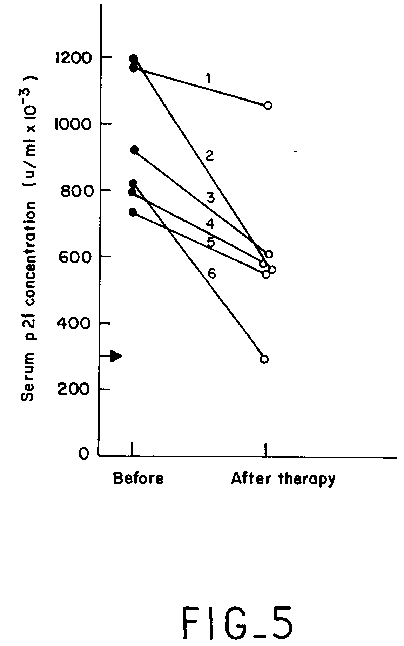

In Figure 5, it is shown the effect of therapy on serum non-**ras**-p21 levels. The arrow indicates the upper level of the normal range, while the numbers indicate the case number. The black circles indicate the status before therapy and the blank ones after the therapy (3 to 26 months). Clinical response to therapy was correlated with a decline in serum non-**ras**-p21 levels in all 6 patients. Case No. 6, refers to a 21 year old patient with testis carcinoma. Subsequent to a surgical treatment, the non-**ras**-p21 serum level decreased to the normal range. Case Number 1, refers to a 64 year old patient with prostate carcinoma; stage C, who had apparently been successfully treated by hormone therapy and radiotherapy and who is presently tested for a relapse.

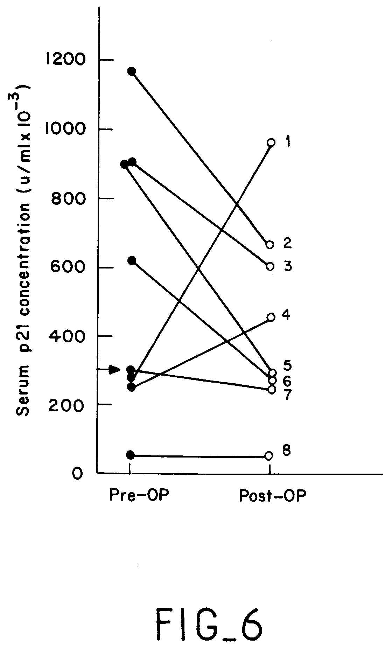

The level of serum non-**ras**-p21 before and after surgery in patients with renal cell carcinoma is shown in Figure 6. The black circles refer to p21 levels before operation and the blank ones after operation (3 to 8 months). The arrow indicates the upper level of the normal range and the case number is indicated at the right hand. Case No. 1 shows a patient with stage III of RCC, in whom lymph node metastasis has been noted and increased non-**ras**-p21 serum level was observed after surgery.

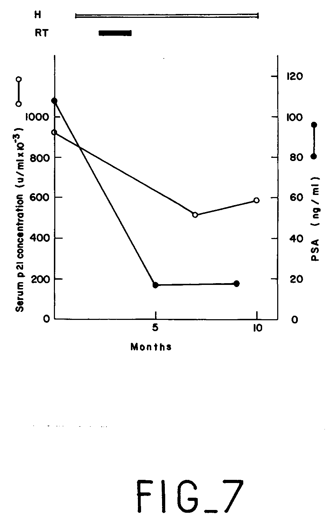

The amount of non-**ras**-p21 is correlated with the level of prostate-specific antigen (PSA) an established tumor marker, as shown in Figure 7.

Among the advantages of the method according to the present invention it should be pointed out that by monitoring serial non-**ras**-p21 levels pre-treatment and post-treatment during the follow-up of cancer patients, it may be possible to provide evidence of response to treatment and recurrence of the disease.

The calibration curve of non-**ras**-p21 to be used in the modified ELISA procedure according to the present invention, is presented in Figure 3. The data for the curve were obtained as follows:

The non-**ras**-p21 protein pellet was dissolved in phosphate bufferred saline (PBS) having a pH of 7.4, at a concentration of 10 $\mu$g/ml. The protein content was determined with Coomassie blue using the method described by Bradford M.M. (Anal. Biochem.72, 248- 254, 1976) by the Bio-Rad Protein Assay and bovine serum albumin as standard. Increasing concentrations of protein: 0; 25; 50; 75; 100; 125; 150; 200 and 300 ng/ml in 100 $\mu$l coating buffer (25 mM $Na_2CO_3$, 55mM $NaHCO_3$, 1mM PMSF having a pH of 9.6, were incubated overnight at 4°C in a polyvinyl microtiter plate (Costar). Since only part of the p21 protein coated the plate and some part was washed out during the ELISA procedure, the amount of 100 ng/ml purified p21 protein was defined as 1 arbitrary unit of protein. This protein served as a positive control for the modified ELISA test. The calibration curve was linear from zero to 150 ng/ml (1.5 arbitrary units) of purified non-**ras**-p21 protein.

The modified ELISA test for a quantitative determination of p21 protein in human serum according to the present invention was carried out as follows:

An amount of 1$\mu$l of serum was diluted with 4 ml of the buffer used in the calibration curve (with the composition as mentioned above). An amount of 100 $\mu$l of serially diluted serum (1/4000 - 1/128000) was applied in duplicate or triplicate to a 96 well U shaped flexible plastic micro-titer plate (Costar) at 4°C, overnight. Each well was subsequently washed twice with phosphate buffered saline (pH 7.4). The plates

8

were coated and kept at room temperature for 90 minutes with a phosphate buffered saline solution containing 2% (weight/volume) nonfat dry milk,and incubated at 37°C for 60 minutes with 100 $\mu$l per well of anti-non-**ras**-p21 antibodies at 1:1000-1/10000 dilution in said buffer containing the nonfat milk. The wells were washed three times with 200 $\mu$l/well of TBST (50 mM Tris pH8,150 mM NaCl plus 0.05% polyoxyethylenesorbitan (Tween 20, Trade Mark of Sigma).The second antibody, biotinylated goat anti-rabbit IgG (produced by Sigma) at a concentration 1:1000-1:2000 was added for 30 minutes at room temperature. The plate was washed three times with TBST. Finally, ExtrAvidin Peroxidase (produced by Sigma), at a concentration of 1:500-1:1000 in TBST,was added for 30 minutes at room temperature and the plate was washed three times with TBS (50mM Tris-HCl, pH = 8.0 and 150 mM sodium chloride). After the final wash, the reaction was developed with a substrate solution (100 $\mu$l per well) containing 0.05% of o-phenylenediamine and 0.05% hydrogen peroxide in a solution of 0.1M phosphate/citrate buffer having a pH of 5.0.After 10 to 30 minutes, the reaction was stopped by the addition of 50 $\mu$l of 2N of sulfuric acid and the colour yield was assessed at 492 nm using the Anthos Labtec 2001 reader. Negative controls included buffer instead of sera to measure non specific binding of the avidin biotinylated peroxidase complex. These negative controls were around 0.1-0.2 A492. Negative controls also included normal human serum (produced by Sigma) which yielded a reading of 0.3 at 492 nm at a dilution of 1/4000. Each point was determined in duplicate, the coefficient of variation being in the range of 3.6% - 10%. Serum non-**ras**-p21 values were calculated from the linear part of the titration curve.

According to another embodiment of the present invention, the detection of the non-**ras**-p21 protein was carried out using the immunoblotting procedure consisting of the following steps:

In a first step, the proteins were separated by electrophoresis on sodium-dodecyl-sulfate/15% polyacrylamide minigels under reducing conditions. The proteins were transferred electrophoretically from the gels to nitrocellulose sheets. The wet membranes were blocked with a solution of 2% (W/V) of nonfat dry milk in a solution of phosphate buffered saline at a pH of 7.4.

The second step consists in the immunodetection of non-**ras**-p21 on nitrocellulose blot. The blots were incubated overnight at 4°C in anti-non-**ras** p21 polyclonal antibodies diluted 1:1000 in blocking solution, or in a normal rabbit serum diluted 1:1000.Antibody binding was detected with horseradish peroxidase-Protein A conjugate (produced by Sigma). The nitrocellulose filter was washed and subsequent color develoment was done with a solution of hydrogen peroxide and 3,3-diaminobenzidine tetrahydrochloride. Figure 1,represents the immunoreactivity of non-**ras** anti-p21 antibodies with non-**ras**-p21, wherein the arrow indicates the non-**ras**-p21 band.

While the invention has been described in respect to some preferred embodiments, it should be understood that a person skilled in the art may introduce various changes and modifications without departing from the underlying principles involved. The invention should not be limited only to the specific details illustrated and described, but rather to the appended Claims.

SEQUENCE LISTING

(1) GENERAL INFORMATION:

    (i) APPLICANT: TECHNION RESEARCH & DEVELOPMENT FOUNDATION Ltd.

    (ii) TITLE OF INVENTION: Novel antibodies useful for the detection of benign and malignant tumors.

    (iii) NUMBER OF SEQUENCES: 2

    (iv) CORRESPONDENCE ADDRESS:
        (A) ADDRESSEE: Cabinet Beau de Lomenie
        (B) STREET: 55, Rue d'Amsterdam
        (C) CITY: Paris
        (E) COUNTRY: FRANCE
        (F) ZIP: 75 008

    (v) COMPUTER READABLE FORM:
        (A) MEDIUM TYPE: Floppy disk
        (B) COMPUTER: IBM PC compatible
        (C) OPERATING SYSTEM: PC-DOS/MS-DOS
        (D) SOFTWARE: PatentIn Release #1.0, Version #1.25

    (vii) PRIOR APPLICATION DATA:
        (A) APPLICATION NUMBER: IL 099630
        (B) FILING DATE: 02-OCT-1991

    (viii) ATTORNEY/AGENT INFORMATION:
        (A) NAME: PORTAL, Gerard
        (C) REFERENCE/DOCKET NUMBER: GPO.JGH/J6772-14

    (ix) TELECOMMUNICATION INFORMATION:
        (A) TELEPHONE: 42 80 64 68
        (B) TELEFAX: 48 74 37 60
        (C) TELEX: 650476F

(2) INFORMATION FOR SEQ ID NO:1:

    (i) SEQUENCE CHARACTERISTICS:
        (A) LENGTH: 15 amino acids
        (B) TYPE: amino acid
        (D) TOPOLOGY: linear

```
(ii) MOLECULE TYPE: peptide

(xi) SEQUENCE DESCRIPTION: SEQ ID NO:1:


Ala Val Gly Asp Arg Arg His Glu Arg Asn Glu Phe Gln Gln Gln
1               5                   10                  15


(2) INFORMATION FOR SEQ ID NO:2:


    (i) SEQUENCE CHARACTERISTICS:
        (A) LENGTH: 12 amino acids
        (B) TYPE: amino acid
        (D) TOPOLOGY: linear


    (ii) MOLECULE TYPE: peptide


    (xi) SEQUENCE DESCRIPTION: SEQ ID NO:2:


Ala Val Ser Pro Ser Pro Thr Phe Leu Thr Gln Gln
1               5                   10
```

**Claims**

1. Novel anti-non-**ras**-p21 antibodies obtained from a p21 protein having a molecular weight of 21kDa and possessing the following N-terminal amino-acid sequence of two peptides derived from the p21 protein:

```
    - Peptide 1 (SEQ ID NO:1) :
    Ala Val Gly Asp Arg Arg His Glu Arg Asn Glu Phe Gln Gln Gln
    1               5                   10                  15



    - Peptide 2 (SEQ ID NO:2) :
    Ala Val Ser Pro Ser Pro Thr Phe Leu Thr Gln Gln
    1               5                   10
```

2. The novel anti-non-**ras**-p21 antibodies according to Claim 1, which reacts with biological fluids of patients with tumors.

3. The novel anti-non-**ras**-p21 antibodies according to Claim 2, wherein said tumors relate to urogenital,gastro-intestinal, lung breast and lymphomas.

4. A method for the detection of benign and malignant tumors in biological fluids of patients which comprises the steps of:
   (a) isolation of 21kDalton proteins from a biological fluid;
   (b) preparation of anti-non-**ras**-p21 polyclonal antibodies which do not possess immunoreactivity with other proteins from the biological fluid, and

(c) determining the total non-**ras**-p21 concentration in biological fluids using a modified ELISA procedure.

5. The method according to Claim 4, wherein in step (a) the proteins are probed by an immunoblotting procedure.

6. The method according to Claim 4, wherein the steps in the modified ELISA procedure are characterized by the following specific features:
   - the serum from the tumor bearing patients is heated at a temperature above 50°C;
   - the calibration curve should be linear from zero to 150 ng/ml;
   - the microtiter plate has to be blocked with a 2% (by volume) of low fat milk in phosphate buffered saline, and
   - the reaction should be stopped by the addition of sulfuric acid.

7. The method according to Claim 4, wherein the monitoring of non-**ras**-p21 levels is useful in pre-treatment and post-treatment during the follow-up of patients bearing malignant tumors.

8. The method according to Claim 7, wherein significant increased levels of p21 are detected in patients with benign prostatic hypertrophy compared with healthy individuals.

FIG_1

FIG_2

FIG_3

FIG_4

FIG_5

FIG_6

FIG_7